(19) Europäisches Patentamt
European Patent Office
Office européen des brevets

(11) EP 3 915 538 A1

(12) # EUROPEAN PATENT APPLICATION
published in accordance with Art. 153(4) EPC

(43) Date of publication:
**01.12.2021 Bulletin 2021/48**

(21) Application number: **20744654.3**

(22) Date of filing: **21.01.2020**

(51) Int Cl.:
***A61K 9/00*** *(2006.01)* ***A61K 9/19*** *(2006.01)*
***A61K 47/36*** *(2006.01)* ***A61K 47/40*** *(2006.01)*
***A61K 47/38*** *(2006.01)* ***A61K 47/32*** *(2006.01)*
***A61K 47/10*** *(2017.01)* ***A61K 31/4412*** *(2006.01)*
***A61P 31/04*** *(2006.01)*

(86) International application number:
**PCT/KR2020/001033**

(87) International publication number:
**WO 2020/153723 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.01.2019 KR 20190007288**

(71) Applicant: **Crystalgenomics, Inc.**
**Seongnam-si, Gyeonggi-do 13488 (KR)**

(72) Inventors:
- **CHO, Jae Pyoung**
  **Gunpo-si, Gyeonggi-do 15822 (KR)**
- **CHO, Joong Myung**
  **Seoul 05553 (KR)**

(74) Representative: **Schön, Christoph**
**Dr. Schön, Neymeyr & Partner mbB**
**Bavariaring 26**
**80336 München (DE)**

(54) **INJECTION COMPOSITION CONTAINING FAB I INHIBITOR, AND PREPARATION METHOD THEREFOR**

(57) The present invention relates to a pharmaceutical composition for intravenous administration, containing a Fab I inhibitor, and a preparation method therefor. The present invention can be effectively applied to an infection caused by antibiotic-resistant bacteria. Specifically, the present invention enables treatment effects to be more rapidly initiated by improving solubility and dissolution rate, and enables bioavailability to be improved. In addition, by controlling the size of particles, mixing and content uniformity of a preparation can be improved.

[Fig. 1]

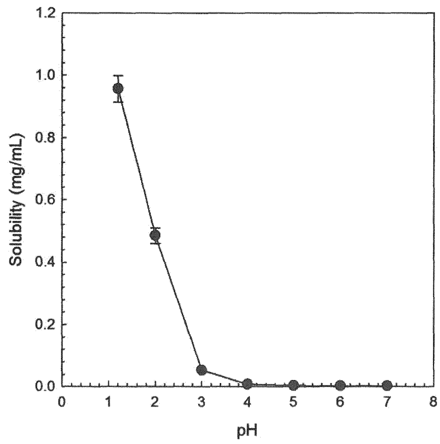


EP 3 915 538 A1

## Description

### Technical Field

The present invention relates to a composition for injection containing a compound that inhibits Fab I or a salt thereof, and a method for preparing the same.

This application claims the benefit of priority to Korean Patent Application No. 2019-0007288, filed on January 21, 2019, the entire disclosure of which is incorporated herein by reference.

### Background Art

Infectious diseases caused by bacteria are diseases that have plagued humans for as a long time as human history and have been a great influence on human history, such as the Black Death. In order to overcome these threats from bacteria, humans have made ceaseless efforts, which has led to the rapid development of medicals and medicines. The development of the modern concept of antibiotics began in 1928 with penicillin, first discovered by Alexander Fleming. Since then, the development of antibiotics to treat bacterial infections had made a leap forward. However, the resistance of the bacteria itself to antibiotics began to be known, and the use of antibiotics was restricted.

Thereafter, as the development of novel antibiotics and the continuous appearance of resistant bacteria against them have been repeated, the development of the novel antibiotics has become a necessary task for the treatment of bacterial infections. In addition, research strategies are also being changed to overcome resistant bacteria. Interest is focused on the development of antibiotics having a new mechanism of action because the resistance that has already been expressed cannot be overcome even though new antibiotics with better efficacy is developed using the previously established bacterial inhibitory mechanism of action. In addition, large pharmaceutical companies such as Bayer, Bristol-Myers Squibb, Merck, Glaxo Smith Kline and Astrazeneca around the world are making great efforts to develop a new concept of antibiotics that can overcome resistance through a completely different mechanism of action from conventional antibiotics. Among these resistant strains, one of the most difficult strains to be treated is MRSA (Methicillin-Resistant Staphylococcus Aureus). MRSA is a staphylococcus aureus that is resistant to methicillin, a penicillin antibiotic. It is not only resistant to methicillin, but has strong resistance to most antibiotics, so it is a pathogen that can be treated only with very limited antibiotics. More than 700,000 people die every year worldwide due to the occurrence of MRSA infection, and the death rate is expected to increase steadily every year, exceeding 10 million by 2050. The reason this strain is attracting attention is not only because it is resistant to existing antibiotics, but also it is the most frequent causative organism among pathogens that induce in-hospital infection and can be fatal to patients with weak immunity or to the old and infirm. In recent years, not only healthcare-acquired MRSA infections but also community-acquired MRSA infections are increasing significantly, indicating that exposure to MRSA occurs easily in everyday life. Vancomycin has been used for its treatment. However, strains resistant to vancomycin have been reported. Other therapeutic agents include Linezolid and Daptomycin, but the selection of antibiotics for therapeutic purpose is very limited.

Therefore, there is an urgent need to develop novel antibiotics that can be applied to antibiotic resistant bacterial infections.

### Detailed Description of the Invention

### Technical Problem

In order to solve the above problems, the present invention provides a composition for injection comprising a Fab I inhibitor, 1-(3-amino-2-methylbenzyl)-4-(2-thiophen-2-yl-ethoxy)-1H-pyridin-2-one or a salt thereof as an active ingredient.

In addition, it provides a method for preparing the composition for injection.

### Solution to Problem

The present invention provides a composition for intravenous injection comprising 1-(3-amino-2-methylbenzyl)-4-(2-thiophen-2-yl-ethoxy)-1H-pyridin-2-one or a salt thereof together with a polymer compound, a solubilizing agent, or a mixture thereof.

According to an embodiment, the content of 1-(3-amino-2-methylbenzyl)-4-(2-thiophen-2-yl-ethoxy)-1H-pyridin-2-one or a salt thereof may be 0.1 to 10% by weight, the content of the polymer compound may be 5 to 40% by weight, and the content of the solubilizing agent may be 10 to 30% by weight.

According to an embodiment, the polymer compound may comprise one or more selected from the group consisting of dextrin, polydextrin, cyclodextrin, poloxamer, dextran, pectin and pectin derivatives, alginate, starch, hydroxypropyl methylcellulose, hydroxypropyl cellulose, hydroxymethyl cellulose, hydroxylethyl cellulose, methylcellulose, sodium carboxymethyl cellulose, hydroxypropyl methylcellulose acetate succinate, hydroxylethylmethyl cellulose, guar gum, locust bean gum, tragacantha, carrageenan, acacia gum, arabic gum, gellan gum, xanthan gum, gelatin, casein, polyvinyl alcohol, polyvinyl pyrrolidone, polyvinylacetaldiethylaminoacetate, poly(butylmethacrylate,(2-dimethylaminoethyl)methacrylate,methylmethacrylate) copolymer, polyethylene glycol, polyethylene oxide and carbomer.

According to an embodiment, the solubilizing agent may comprise one or more selected from the group consisting of propylene glycol, polyethylene glycol, dipropylene glycol, diethylene glycol, diethylene glycol monoethyl ether, glycerol, Tween 80, cremophor and transcutol.

According to an embodiment, the composition may be provided as an injection in the form of a liquid, emulsion or lyophilized powder.

According to another embodiment, the present invention provides a method of preparing a composition for injection comprising 1-(3-amino-2-methylbenzyl)-4-(2-thiophen-2-yl-ethoxy)-1H-pyridin-2-one or a salt thereof, the method comprising:

adding 4-benzyloxy-1H-pyridone, 2-methyl-3-nitro-benzylchloride and potassium tert-butoxide to dimethylformamide and mixing and reacting them under heating;
reacting the mixture, adding purified water and drying under heating;
dissolving the dried product in an organic solvent and adding purified water for layer separation;
recovering the organic layer, filtering and concentrating to prepare a concentrate;
reconcentrating the concentrate and adding hexane to prepare an intermediate precipitate;
dissolving the obtained precipitate, cooling, filtering and drying to obtain a dried product; and
dissolving the obtained dried product in an organic solvent and then adding and reacting iron chloride hexahydrate, activated carbon and hydrazine monohydrate, cooling and filtering to obtain a final precipitate, and drying and pulverizing the obtained final precipitate to prepare the compound.

According to an embodiment, the method may further comprise adding an acidic substance.

According to an embodiment, the acidic substance may comprise one or more selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, oxalic acid, fumaric acid, malonic acid, maleic acid, methanesulfonic acid, benzenesulfonic acid, p-toluenesulfonic acid, naphthalenesulfonic acid and EDTA.

According to an embodiment, the method may further comprise:
dissolving a polymer compound and 1-(3-amino-2-methylbenzyl)-4-(2-thiophen-2-yl-ethoxy)-1H-pyridin-2-one or a salt thereof in a solvent; and vacuum drying the solution and then micronizing the resulting solid to prepare a polymer dispersion.

According to an embodiment, the method may further comprise dissolving a polymer compound and a lipid-based surfactant and 1-(3-amino-2-methylbenzyl)-4-(2-thiophen-2-yl-ethoxy)-1H-pyridin-2-one or a salt thereof in a solvent;

gradually adding a solubilizing agent to the solution; and
centrifugating and vacuum drying the solution and then homogenizing to prepare a liposome formulation.

According to an embodiment, the lipid-based surfactant may include soybean oil.

According to an embodiment, the composition may be prepared in the form of a solid dispersion, a liposome formulation, or a combination thereof.

According to an embodiment, the composition may be used for the treatment of bacterial infections.

Other specifics of the embodiments of the present invention are included in the detailed description below.

## Effect of the Invention

The composition for injection comprising a Fab I inhibitor of the present invention or a salt thereof can be effectively applied to infections caused by antibiotic-resistant bacteria. Specifically, the present invention improves the solubility of a Fab I inhibitor or a salt thereof having a significantly low solubility, and improves storage stability, thereby allowing intravenous administration, so that the therapeutic effect can be initiated more quickly.

## Brief Description of Drawings

Fig. 1 is a graph showing the solubility of a compound of formula 1 depending on pH.
Fig. 2 is a photograph of observing the appearance of an undiluted liposome formulation and the appearance of a liposome formulation after dilution in water for injection.
Fig. 3 is microscopic observation photograph of liposome formulations according to Examples 3 to 5.
Fig. 4 is a schematic diagram showing a method for preparing a cyclodextrin inclusion compound.
Fig. 5 is a graph showing the antibacterial effect according to the T/MIC value.

**Best Mode for Carrying out the Invention**

Since various modifications and variations can be made in the present invention, particular embodiments are illustrated in the drawings and will be described in detail in the detailed description. It should be understood, however, that the invention is not intended to be limited to the particular embodiments, but includes all modifications, equivalents, and alternatives falling within the spirit and scope of the invention. In the following description of the present invention, detailed description of known functions will be omitted if it is determined that it may obscure the gist of the present invention.

Hereinafter, the injection composition according to an embodiment of the present invention will be described in more detail.

The term "pharmaceutical composition" as used herein may be described interchangeably with "pharmacological composition" and "pharmaceutically acceptable composition" and refers to any composition which can be a relatively non-toxic to a subject to be administered and have harmless effective action. In addition, it may refer to any organic or inorganic compound formulation in that side effects resulting from the composition do not impair the efficacy of the drug, and that does not cause serious irritation to a subject to be administered by the compound and does not impair the biological activities and properties of the compound.

As used herein, the term "subject to be administered" may be used interchangeably with "individual to be administered" and "organism to be administered" and may refer to any animals including humans in which infection with bacteria or resistant strains is caused or may be caused.

In addition, the term 'bacterial infection' may be used interchangeably with 'bacteria-related disease', and refers to a disorder or disease caused by bacterial infection. The disorder or disease may include, for example, urinary tract, respiratory or skin tissue infection, sepsis, and the like, but is not limited thereto.

The present invention provides a composition for injection comprising a Fab I inhibitor. Specifically, the composition of the present invention may comprise 1-(3-amino-2-methylbenzyl)-4-(2-thiophen-2-yl-ethoxy)-1H-pyridin-2-one, a salt thereof or a combination thereof as a selective Fab I inhibitor.

The structure of 1-(3-amino-2-methylbenzyl)-4-(2-thiophen-2-yl-ethoxy)-1H-pyridin-2-one is represented by the following formula 1.

[Formula 1]

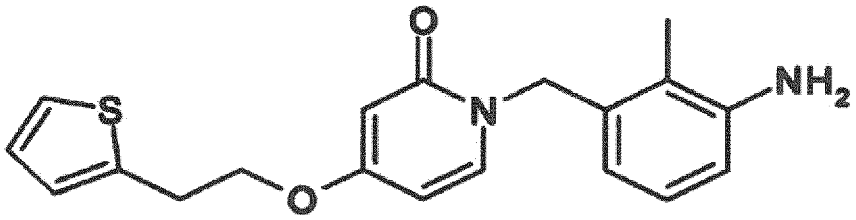

Chemical name: 1-(3-amino-2-methylbenzyl)-4-(2-thiophen-2-yl-ethoxy)-1H-pyridin-2-one

The selective Fab I inhibitor, for example, having the structure of formula 1 is a compound that has a completely different mechanism of action from the existing antibiotics, such as beta-lactam antibiotics (penicillin, cephalosporin, etc.), glycopeptides (vancomycin, etc.), tetracyclines, aminoglycosides, glycylclines, macrolides, chloramphenicol, quinolones, sulfonamides, and oxazolines and a substance that exhibits antibiotic efficacy by inhibiting the action of the enzyme Fab I essential for protein synthesis in bacteria.

Fatty acids, which are not only an energy source for living organisms but also a major component of cell membranes, play an essential role in maintaining life phenomena. Therefore, biosynthesis processes of the fatty acids in cells are essential biochemical processes that exist in all living cells. Genes involved in these processes are one of essential genes in from bacteria to humans.

Fab I, which is an enoyl-ACP reductase in the final step of the cycle, among the four enzymes involved in bacterial fatty acid biosynthesis, has been reported to play a role in converting enoyl-ACP to the corresponding acyl-ACP through a 1,4-reduction reaction ((Payne et al., Drug Discovery Today 6, 2001, 537-544). Fab I is the most important

protein in fatty acid synthesis and involved in the reaction that determines the rate of the overall synthesis process. However, in mammals such as humans, unlike bacteria, a huge group of enzymes called fatty acid synthase are used for the synthesis of such fatty acids. Moreover, their structures are completely different from the proteins in the bacterial fatty acid synthesis pathway. Therefore, since a selective Fab I inhibitor has little toxicity and is an inhibitor against a novel target protein that has not been targeted with any antibiotic until now, the development of drugs that act on this target protein can improve a treatment success rate against bacteria having drug resistance, especially multidrug resistance.

According to an embodiment, the compound of formula 1 may be provided in the form of an amorphous form, a crystalline form, or a mixture thereof.

According to another embodiment, the compound of formula 1 may be prepared by the method comprising:

adding 4-benzyloxy-1H-pyridone, 2-methyl-3-nitro-benzylchloride and potassium tert-butoxide to dimethylformamide and mixing and reacting them under heating;
reacting the mixture, adding purified water and drying under heating;
dissolving the dried product in an organic solvent and adding purified water for layer separation;
recovering the organic layer, filtering and concentrating to prepare a concentrate;
reconcentrating the concentrate and adding hexane to prepare an intermediate precipitate;
dissolving the obtained precipitate, cooling, filtering and drying to obtain a dried product; and
dissolving the obtained dried product in an organic solvent and then adding and reacting iron chloride hexahydrate, activated carbon and hydrazine monohydrate, cooling and filtering to obtain a final precipitate, and drying and pulverizing the obtained final precipitate.

In addition, the present invention provides a method for preparing a composition for intravenous injection comprising the compound of formula 1 as described above as a Fab I inhibitor.

According to an embodiment, a pharmaceutically acceptable salt of the compound of formula 1 may be contained in the composition for injection. The pharmaceutically acceptable salt may be an acid addition salt formed using an acid. Examples of the acid include hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, oxalic acid, fumaric acid, malonic acid, maleic acid, methanesulfonic acid, benzenesulfonic acid, p-toluenesulfonic acid, naphthalenesulfonic acid, ethylendiaminetetraacetic acid (EDTA) and the like, but are not limited thereto.

According to one embodiment, the present invention may be formulated in a solid or liquid form capable of intravenous administration.

Specifically, the present invention can provide a formulation in an emulsion or liquid form by remarkably increasing the water solubility of the Fab I inhibitor (the compound of formula 1) by combining the compound of formula 1 or a salt with a solubilizing agent, a co-surfactant, and a lipid. As the solubilizing agent, co-surfactant, and lipid, a non-toxic pharmaceutically acceptable material is used, examples of which include propylene glycol, polyethylene glycol, dipropyleneglycol, diethylene glycol, diethylene glycol monoethyl ether, glycerol, tween 80, cremophor, transcutol, and the like, but are not limited thereto. They may be prepared in an emulsion or liquid form capable of intravenous administration of the composition of the present invention through an appropriate combination. The solubilizing agent may be contained in an amount of 10 to 30% by weight based on the total weight of the composition, for example 10% by weight or more, or 15% by weight or more, or 20% by weight or more and, for example, 30% by weight or less, or 25% by weight or less. The composition capable of intravenous administration may include, for example, an injection.

In addition, according to an embodiment, the composition of the present invention may be made into nanoparticles to increase a surface area or may be enclosed in a porous polymer material to improve solubility.

According to an embodiment, nanoparticle formation involves dissolving and mixing the compound of formula 1 in an appropriate solvent, then rapidly lowering the temperature to generate a solid, pulverizing it with a pulverizer, and supercritical extraction to obtain a product.

According to an embodiment, as the polymer material, water-soluble polymers may be used and examples thereof include dextrin, polydextrin, cyclodextrin, dextran, pectin and pectin derivatives, alginate, starch, hydroxypropyl methycellulose, hydroxypropyl cellulose, hydroxymethyl cellulose, hydroxylethyl cellulose, methyl cellulose, sodium carboxymethyl cellulose, hydroxypropyl methylcellulose acetate succinate, hydroxylethylmethyl cellulose, guar gum, locust bean gum, tragacantha, carrageenan, acacia gum, arabic gum, gellan gum, xanthan gum, gelatin, casein, polyvinyl alcohol, polyvinyl pyrrolidone, polyvinylacetaldiethylaminoacetate, poly(butylmethacrylate,(2-dimethylaminoethyl)methacrylate,methylmethacrylate) copolymer, polyethylene glycol, polyethylene oxide, carbomer, and the like, which may be used alone or in combination of two or more. It may be contained in an amount of 5 to 40% by weight, for example 10 to 20% by weight, for example 5% by weight or more, or 10% by weight or more and 40% by weight or less, or 35% by weight or less, or 30% by weight or less, or 25% by weight or less, or 20% by weight or less based on the total weight of the composition.

According to an embodiment, the composition of the present invention may be prepared in a solid form of lyophilized powder, thereby further improving solubility and storage stability. For example, the composition may be prepared and commercialized in the form including nanoparticles, hydrophilic polymer compounds, or combinations thereof.
According to an embodiment, the compound of formula 1, a salt thereof, or a combination thereof may be contained in an amount of 0.1 to 10% by weight, for example 0.2 to 2% by weight, for example 0.1% by weight or more, or 0.2% by weight or more, or 0.5% by weight or more, and for example 10% by weight or less, or 8% by weight or less, or 5% by weight or less, or 2% by weight or less based on the total weight of the composition.
According to an embodiment, the composition in a powder form may be dissolved in water for injection prior to application to a subject to be administered. The water for injection includes, for example, glucose, xylitol, D-mannitol, fructose, physiological saline, dextran 40, dextran 70, amino acids, Ringer's solution, lactic acid-Ringer's solution, and the like, but is not limited thereto.
According to an embodiment, the present invention can be used for the treatment of gram-positive bacterial infections such as MRSA (methicillin resistant staphylococcus aureus) or various infectious diseases thereof. Gram-positive bacteria include, for example, *Staphylococcus*, such as *Staphylococcus aureus* and *Staphylococcus epidermidis*; and *Streptococcus*, such as *Streptococcus pneumonia*, *Streptococcus pyrogenes*, group C/F/G *Streptococci* and viridans group *Streptococci.*
Hereinafter, embodiments of the present invention will be described in detail so that those skilled in the art can easily carry out the present invention. The present invention may, however, be embodied in many different forms and should not be construed as limited to the embodiments set forth herein.

Preparation Example 1: Preparation of compound of formula 1

To prepare the compound of formula 1, 0.9 mol of 4-benzyloxy-1H-pyridone was introduced into 10 L of dimethylformamide (DMF) and then 0.9 mol of potassium tert-butoxide was added thereto with stirring. It was warmed to 55 °C with stirring for 30 minutes. 0.9 mol of 2-methyl-3-nitrobenzyl chloride was slowly added and reacted while mixing for an additional 2 hours. After the reaction was completed, 4 L of purified water was added thereto, followed by drying in a rotary evaporator (Rotavapor® R-220, BUCHI) while heating to 60 °C. 14 L of dimethyl chloride was added to dissolve the dried product and 7 L of distilled water was added for layer separation. A supernatant was taken and then 0.7 kg of each of magnesium sulfate and activated carbon were added to the supernatant, and the solution was stirred for 1 hour, filtered through Celite, and dried using a rotary evaporator (yield: 60%).
After dissolving about 2 kg of the resulting dried product in ethanol, 100 g of iron chloride hexahydrate, 600 g of activated carbon, and 10 kg of hydrazine monohydrate were added to react and the resulting solution was cooled. Thereafter, it was filtered to obtain white precipitates, which were dried overnight at 40 °C in a vacuum oven to produce a pyridine substituent, 1-(3-amino-2-methylbenzyl)-4-(2-thiophen-2-yl-ethoxy)-1H-pyridin-2-one, which is a compound of formula 1. The yield was 85%.
In order to remove the related substances generated in the synthesis process, purification may be performed if necessary. Purification is carried out as follows: 2 kg of the synthesized raw material is dissolved in 30 L of dichloromethane and then purified water is added for layer separation. The organic layer is taken, and then 0.7 kg of sodium sulfate is added and additionally 0.7 kg of activated carbon is added to the organic layer. The solution is stirred for 1 hour, filtered and concentrated under reduced pressure to remove dichloromethane. Further, it is dissolved by adding 10 L of ethyl acetate, concentrated, and recrystallized by adding 20 L of hexane, and then dried at 40 °C. The purification operation can be repeated as needed.

Experimental Example 1: Evaluation of solubility

Experimental Example 1-1: Evaluation of water solubility according to pH

Solubility according to pH change was measured for the compound of formula 1 (1-(3-amino-2-methylbenzyl)-4-(2-thiophen-2-yl-ethoxy)-1H-pyridin-2-one). To this end, an excess of the compound of formula 1 was added to aqueous solutions having a different pH value from pH 1.2 to 7.0, respectively, followed by stirring at room temperature for 2 hours.
After completion of the stirring, insoluble substances that may remain were removed by first centrifugation and second filtration with 0.22 μm filter. The substances were diluted with organic solvent methanol and analyzed by HPLC for qualification of the solubility. The results are shown in Fig. 1. As shown in Fig. 1, it can be seen that the solubility of the compound of formula 1 is affected by the pH of the aqueous solution. Specifically, it exhibits a solubility of about 1 mg/ml at pH 1.2, but 6 μg/ml at pH 3 and 2.5 μg/ml at pH 7. As the pH increases, the solubility tends to decrease rapidly. In general, the solubility in pH 4 to 8, which is the pH range suitable for intravenous administration, shows a very low value of 2 to 5.0 μg/mL, and therefore, a research for increasing the solubility as a Fab I inhibitor is required.

Experimental Example 1-2: HPLC analysis

The excipients approved as pharmaceutical additives and solvents that can be used for future process studies were selected as shown in Table 1. A small amount of the compound of formula 1 was added and dissolved. The addition operation was repeated until completely dissolved. Finally, it was stirred at room temperature for one hour and then filtered to determine the concentration of the compound of formula 1 dissolved in the filtrate through HPLC analysis.

20 μL of each the test solution and the standard solution were tested according to a liquid chromatography method (HPLC) of general test methods of the Korean Pharmacopoeia under the following conditions, and the peak area of the main component of each solution was measured.

Operating condition and calculation

[Operating condition]

Detector: UV spectrophotometer (measurement wavelength 286 nm)
Column: Aegispak C18-L (4.6 mm x 250 mm, 5 μm) column
Column temperature: 25 °C
Mobile phase: Acetonitrile: water = 3: 2
Flow rate: 1.0 mL/min

[Calculation]

$$\text{Content (\%)} = \frac{A_T}{A_S} \times \frac{D_T}{D_S} \times P$$

$A_T$ : Peak area of the main component in the test solution
As : Peak area of the main component in the standard solution
$D_T$ : Dilution factor of the test solution
Ds : Dilution factor of the standard solution
P: Purity of the main component standard product (%)

[Table 1]

| Chemicals | Solubility (mg/mL) | Chemicals | Solubility (mg/mL) |
|---|---|---|---|
| Water | 0.0025 (2.5 μg/mL) | Soybean Oil | 8.5 |
| pH2.0 | 0.44 | Capryol 90 | 2.9 |
| Ethanol | 3.65 | Oleic acid | 2 |
| Methanol | 6.14 | Peceol | 4.4 |
| Glycerol | 0.26 | Tween 80 | 10.2 |
| Methyl chloride | 60 | Solutol HS 15 | 12.9 |
| DMSO | 180 | Tween 20 | 30.7 |
| PEG 300 | 22 | Labrasol | 30.8 |
| Propylene glycol | 3.8 | - | - |

As shown in Table 1, the organic solvent showed a solubility of about 60 mg/mL in methylene chloride, and more than 20 mg/mL in PEG300, Tween 20 and Labrasol. Excipients with a solubility of 10 mg/mL or more were Tween 80 and Solutol HS 15, and soybean oil, among the Lipid-based, showed a solubility of about 8.5 mg/mL. Among the selected drugs, Tween 20, Tween 80, Solutol HS 15, Soybean oil and PEG 300 can be used for injection. Therefore, selected excipients were used for future studies, and in particular, a study on the injection formulation was conducted

using a combination thereof.

Experimental Example 2: Evaluation of excipient suitability

As shown in Table 2, an excipient licensed for medicine and the compound of formula 1 were mixed and dissolved in methylene chloride, an organic solvent, and dried in vacuum to volatilize the added organic solvent. The resulting product was stored at 60 °C and the stability of the compound of formula 1 was measured. The measurement of related substances was evaluated according to the guidelines for the evaluation of related substances of the Ministry of Food and Drug Safety, and the results are shown in Table 2.

[Table 2]

| Chemicals | Total related substances | | Chemicals | Total related substances | |
|---|---|---|---|---|---|
| | Initial value | Severe (1 week) | | Initial value | Severe (1 week) |
| Compound of formula 1 | 0.5 | 0.5 | PEG 400 | 6.4 | 9.8 |
| Soybean Oil | 1.1 | 1.4 | PEG 20,000 | 4.2 | 1 |
| Tween 80 | 1.2 | 2.6 | PVA | 0.9 | 0.6 |
| Solutol HS 15 | 0.7 | 3.8 | PVA 2,000 | 0.9 | 0.6 |
| Propylene glycol | 1 | 5 | Poloxamer 407 | 0.9 | 0.5 |
| Transcutol HP | 1.9 | 9.1 | Glycerin | 1.4 | 1.1 |
| Cremophor 40 | 1.6 | 3.4 | Oleic acid | 6.2 | - |
| Olive oil | 1.1 | 0.7 | Labrasol | 6.2 | - |
| PEG | 1.1 | 7 | Beta-Hydroxypropyl cyclodextrin | 0.5 | 0.6 |

As shown in Table 2, it was confirmed that the compound of formula 1 is relatively stable with excipients such as soybean oil, olive oil, Tween 80, PEG 20000, PVA, PVA 2000, poloxamer 407, beta-hydroxypropyl cyclodextrin, and glycerin. PVA series is an excipient generally used in eye drops. Considering solubility and compatibility comprehensively, future studies on formulations can be performed using excipients such as soybean oil, Tween 20, Tween 80, poloxamers and PEG 20000.

Example 1: Preparation of polymer dispersion

A polymer dispersion was prepared in order to improve solubility by using PEG 20000 (PEG20K) and poloxamer 407 (P407), which are compatible with the compound of formula 1. To this end, each polymer compound was dissolved in methylene chloride (DCM), and the compound of formula 1 was added and dissolved, followed by vacuum drying. The resulting solid was pulverized and then micronized. The composition of the prepared composition is shown in Table 3.

[Table 3]

| Chemicals | Unit | PEG20K: Compound of formula 1 | PEG20K: Compound of formula 1 | PEG20K: Compound of formula 1 | P407: Compound of formula 1 | P407: Compound of formula 1 | P407: Compound of formula 1 |
|---|---|---|---|---|---|---|---|
| | | 20:1 | 10:1 | 5:1 | 20:1 | 10:1 | 5:1 |
| Compound of formula 1 | 9 | 0.1 | 0.1 | 0.1 | 0.1 | 0.1 | 0.1 |
| PEG 20000 | 9 | 2 | 1 | 0.5 | - | - | - |
| Poloxamer 407 | 9 | - | - | - | 2 | 1 | 0.5 |
| DCM | ml | 10 | 10 | 10 | 10 | 10 | 10 |
| Appearance after drying | | Solid | Solid | Solid | Solid | Solid | Solid |
| HLB value | | 18.3 | 17.7 | 16.7 | 19.3 | 18.6 | 17.5 |
| Solubility | | Well dissolved | X | X | Well dissolved | X | X |
| Feature | | Precipitation occurs within 10 minutes after dissolving | Dissolved when ultrasonic waves are applied, precipitation occurs | Dissolved when ultrasonic waves are applied, precipitation occurs | Precipitation occurs within 30 minutes after dissolving | Dissolved when ultrasonic waves are applied, precipitation occurs | Dissolved when ultrasonic waves are applied, precipitation occurs |

As shown in Table 3, as the content of the polymer compound in the polymer dispersion increased, the compound of formula 1 was generally well dissolved. In particular, when the content ratio of PEG 2000 or poloxamer 407 to the compound of formula 1 was 20:1, the maximal solubility was measured to be about 12 mg/mL (12,000 μg/mL) and 15 mg (15,000 μg/ml), respectively. As shown in Table 3, the actual concentration of the injection formulation prepared at a content ratio of 20:1 of each substance was 10 mg/mL, which is lower than the maximal solubility. The concentration of 10 mg/mL of this formulation is approximately 4,000 times as compared to 2.5 μg/mL of water solubility of the compound of formula 1 .

However, when diluted 50- to 100-fold for IV infusion injection, precipitation occurred within 10 to 30 minutes. In addition, the precipitation rate when the compound of formula 1 combined with poloxamer 407 was dissolved in water for injection was decreased compared to when PEG 20,000 was used. This is because aggregation due to hydrophobic properties of the Fab I inhibitor (compound of formula 1) present in the solution occurs, resulting in crystallization. Since poloxamer is more hydrophobic than PEG 20,000, it is thought that precipitation occurs slowly because it can form hydrophobic bond with the compound of formula 1. The molecular weight of the compound of formula 1 is 340.45, and the hydrophile-lipophile balance (HLB) value is determined by multiplying the ratio of the molecular weight of the hydrophilic portion of the molecule to the molecular weight of the whole molecule weight by 5. The HLB value of the compound of formula 1 thus calculated is 5.4. The HLB values of PEG 20,000 and poloxamer 407 are approximately 19 and 20. Therefore, the HLB value of each composition is calculated by multiplying the weight fraction of each substance by the total sum of the HLB values. The results are shown in Table 3. As the content of the compound of formula 1 was increased, the HLB value was decreased and the hydrophobic properties of the polymer dispersion was increased. Thus, the dissolution did not occur easily, and when the dissolution occurred by applying ultrasonic waves, nanoparticles with a slightly bluish tint were formed, but precipitation of the compound of formula 1 was observed within about 30 minutes. As a result, it was confirmed that when the polymer dispersion was applied, the solubility could be increased overall due to change of a crystalline structure of the compound of formula 1 to an amorphous structure.

Examples 2 to 5: Preparation of liposome formulation

In order to block the hydrophobic bonding of the compound of formula 1 by adding a substance with stronger hydrophobic properties to the polymer dispersion, a microemulsion or liposome was prepared. A drug was embedded in the particles to prevent occurrence of precipitation due to hydrophobic bonds.

In order to improve the solubility of the poorly soluble compound of formula 1, a study on the formulation was conducted by using manufacturing technologies of polymer dispersion and liposome as above-mentioned in combination. For this, poloxamer 407, which had a slow precipitation rate in the polymer dispersion, was selected. Then, soybean oil was used as a lipid-based surfactant. In the evaluation of solubility of Experimental Example 1-2, 8.5 mg of the compound of formula 1 was dissolved in 1 mL of soybean oil. In the evaluation of the suitability of Experimental Example 2, soybean oil did not affect the stability of the compound of formula 1 relatively much. In addition, it was attempted to control the stability of the microemulsion generated in the solution by using lecithin having a low critical micelle concentration (CMC). Specifically, first, the compound of formula 1 was dissolved in methylene chloride, and a solubilizing agent was gradually added thereto. If the added solubilizing agent was not sufficiently dissolved, methylene chloride was additionally added, resulting in a pale-yellow liquid. The volume of methylene chloride used for dissolution is approximately 15 mL. The solution thus prepared was centrifuged to remove insoluble substances and vacuum dried to remove methylene chloride. Secondary distilled water was added to the vacuum-dried material and homogenized to prepare a liposome.

The specific composition of the liposome formulations (Examples 2 to 5) is shown in Table 4. Fig. 2 is a photograph showing the appearance of the liposome formulation of Example 5 and the appearance of the formulation diluted 20-fold with water for injection (0.9% NaCl solution).

[Fig. 4]

| Chemicals | Example 2 | Example 3 | Example 4 | Example 5 |
|---|---|---|---|---|
| Compound of formula 1 | 0.2 g | 0.2 g | 0.2 g | 0.2 g |
| Soybean oil | 1 g | 1 g | 0.5 g | 1 g |
| Lecithin | 0.02 g | - | - | - |
| Poloxamer 407 | 0.5 g | 0.5 g | 0.5 g | 1 g |
| Tween 80 | 0.5 g | 0.5 g | 0.5 g | 1 g |
| Phosphoric acid (85%) | 1.6 g | - | - | - |
| Water | 7.78 g | 7.8 g | 8.3 g | 6.8 g |

(continued)

| Chemicals | Example 2 | Example 3 | Example 4 | Example 5 |
|---|---|---|---|---|
| Precipitation | × | × | O | × |
| pH | 1.8 | 6.7 | 6.5 | 6.8 |

As shown in Fig. 2, it was found that the prepared composition had an appearance of opaque suspension and was easily diluted in water for injection (0.9% NaCl solution) and it could be easily injected with a 22G injection needle. In addition, as shown in Table 4, it was found that in the case that the content of soybean oil relative to the total weight of the composition decreases, precipitation occurs when left at room temperature for 24 hours. However, it was found that in the case that poloxamer and Tween 80 are added to the formulation, precipitation does not occur when left at room temperature for 24 hours, which means that the physical stability is improved.

In Example 5, the compound of formula 1 was prepared to have a concentration of 20 mg/mL, which is 8,000 times the water solubility of the compound of formula 1 of 2.5 μg/mL.

Since the compound of formula 1 has a low solubility in water, phosphoric acid is used in Example 2. However, a low pH condition of pH 2 or less may cause unexpected side effects such as phlebitis when injected into the body. Therefore, the composition as in Examples 3 to 5 was designed. It was found that when phosphoric acid was removed, a microemulsion was easily formed. In the case of Example 5, the concentration of the compound of formula 1 was 20 mg/mL, as described above, and the undissolved precipitates of the compound of formula 1 was not observed, so it was determined to be completely dissolved.

When observed with the naked eye, the acid-containing formulation had high turbidity when diluted. However, when the acid was removed, a slightly bluish suspension formulation was formed. In general, when the acid was not used, the turbidity was low.

On the other hand, it was confirmed that as the content of soybean oil was increased, the precipitation of the compound of formula 1 was delayed, but the particle size of the resulting microemulsion tended to increase. In Example 5, when the content of Tween 80 and poloxamer 407 was increased, the size of the resulting particles was decreased and unembedded compound of formula 1 was not observed. The microscopic observation photograph is shown in Fig. 3.

In case of applying to injections, the average particle diameter of particles can be adjusted to 5 μm or less in order to improve suitability.

Experimental Example 3: Evaluation of stability of liposome formulation

In order to evaluate the stability of the liposome formulation, the composition of Example 5 was observed for changes in long-term and accelerated conditions, and the conditions and results are shown in Table 5.

[Table 5]

| Period | Condition | Maximal individual related substance (%) | Total related substances (%) | pH | Precipitation |
|---|---|---|---|---|---|
| Initial | - | 0.12 | 0.54 | 6.8 | × |
| 1 month | Long-term, 25 °C, 60%RH | 0.13 | 0.45 | 6.7 | X |
| | Accelerated, 40 °C, 75%RH | 0.14 | 0.57 | 6.9 | X |
| 2 months | Long-term, 25 °C, 60%RH | 0.11 | 0.55 | 6.6 | X |
| | Accelerated, 40 °C, 75%RH | 0.13 | 0.58 | 6.8 | x |
| 3 months | Long-term, 25 °C, 60%RH | 0.1 | 0.52 | 6.5 | X |
| | Accelerated, 40 °C, 75%RH | 0.1 | 0.6 | 6.6 | X |

As shown in Table 5, no significant physical and chemical changes were observed for 3 months in the long-term and accelerated conditions.

Example 6: cyclodextrin complexation

In order to further improve the solubility of the composition, (2-hydroxypropyl)-p-cyclodextrin (HP-beta-cyclodextrin) was used to enclose the compound of formula 1 therein. The specific composition is shown in Table 6, and the manufacturing method is shown in a schematic diagram in Fig. 4.

[Table 6]

| Component | Q'ty/Cap | Function |
|---|---|---|
| CG400549 | 4 mg | Active ingredient |
| PEG 300 | 112 mg | Plasticizer |
| Propylene glycol | 24 | Plasticizer |
| Dehydrated EtOH | 79.2 mg | Solvent |
| Benzyl alcohol | 1.8 mg | Preservative |
| HP-b-CD | 100 mg | Carrier |
| NaCl | 9 mg | Buffer agent |
| Water | q.s. | Solvent |

Referring to Fig. 4, the compound of formula 1 was enclosed in HP-beta-cyclodextrin, mixed with a solution in which a solubilizing agent was dissolved, and stirred until a transparent solution was obtained. Finally, the solution was adjusted to have pH of 3.0 and then diluted with water for injection (0.9% NaCl solution) and filtered. The concentration of the compound of formula 1 in the finally prepared formulation was 4 mg/mL, and no precipitates were observed, so it was judged to be completely dissolved. This is a value that is approximately 1,600 times improved compared to 2.5 µg/mL of the water solubility of the compound of formula 1 at a concentration of 4 mg/mL.

Experimental Example 4: Evaluation of stability of cyclodextrine formulation

In order to evaluate the stability of the cyclodextrin formulation, the composition of Example 6 was observed for changes in long-term and accelerated conditions. Table 7 shows the results of long-term storage and Table 8 shows the results of accelerated storage.

[Table 7]

| Test item | | Specification | Initial value | 1 month |
|---|---|---|---|---|
| Appearance | | Colorless clear liquid | Colorless clear liquid | Colorless clear liquid |
| Content | | 95~ 105% | 104.1 | 102.7 |
| Related substances | Unknown individual related substance | 0.2% | 0.2 | 0.26 |
| | Total related substances | 2.0% | 0.2 | 0.68 |
| pH | | pH 2.5~3.5 | 3.25 | 3.04 |

[Table 8]

| Test item | Specification Initial value 1 month | | |
|---|---|---|---|
| Appearance | Colorless clear liquid | Colorless clear liquid | Colorless clear liquid |
| Content | 95~ 105% | 104.1 | 104.7 |

(continued)

| Test item | | Specification | Initial value | 1 month |
|---|---|---|---|---|
| Appearance | | Colorless clear liquid | Colorless clear liquid | Colorless clear liquid |
| Related substances | Unknown individual related substance | 0.2% | 0.2 | 0.59 |
| | Total related substances | 2.0% | 0.2 | 2.63 |
| pH | | pH 2.5~3.5 | 3.25 | 3.05 |

As shown in Table 7, no significant physical and chemical changes were observed in appearance, content, related substances and pH during the stability test under long-term conditions. However, as shown in Table 8, no significant changes were observed in appearance and content, but the content of unknown individual related substance in related substances was increased to about 0.6%, which exceeded the standard 0.2% when 1 month elapsed under accelerated condition, and also the pH was decreased from 3.25 to 3.05. This shows that the composition prepared according to Example 6 is physicochemically unstable.

Examples 7 to 11: Selection of concentration of compound of formula 1 in cyclodextrin formulation

When a large amount of low molecular weight substances ethanol, PEG 300, propylene glycol, etc., are contained in the composition of Example 6, there is a risk of side effects such as the occurrence of phlebitis when administered directly, due to an increase in osmotic pressure and a low pH of 3.0. Accordingly, the pH was brought to close to neutral and a low molecular weight solubilizing agent was not used to prepare the composition.

Hydroxypropyl beta-cyclodextrin (HP-beta-cyclodextrin) was dissolved in distilled water for injection under stirring at room temperature and heated to 60 °C, and the compound of formula 1 was added thereto, stirred and enclosed therein. The liquid injection was sterilized and filtered through 0.22 μm filter paper. The filtered injection solution was filled into a glass vial, cooled at -80 °C, and freeze-dried to commercialize. The specific composition is shown in Table 9.

[Table 9]

| Item | | Example 7 | Example 8 | Example 9 | Example 10 | Example 11 |
|---|---|---|---|---|---|---|
| Main component | Compound of formula 1 | 5 mg | 10 mg | 30 mg | 50 mg | 100 mg |
| Solubilizing agent | 2-hydroxypropyl cyclodextrin | 1600 mg | 1600 mg | 1600 mg | 1600 mg | 1600 mg |
| Investigation of stability after dilution of prepared freeze-dried powder | | | | | | |
| Diluent | Physiological saline | | | | | |
| Final volume | Unit (mL) | 10 | 10 | 10 | 10 | 10 |
| Concentratio n | Unit (mg/ml) | 0.5 | 1 | 3 | 5 | 10 |
| Precipitation | After 4 hours | Clear | Clear | Clear | Precipitation | Precipitation |
| | After 24 hours | Clear | Clear | Clear | Precipitation | Precipitation |

As shown in Table 9, the highest solubility of compound of formula 1 in 16% HP-beta-cyclodextrin is 3 mg/mL, which is about 1200 times improved solubility compared to 2.5 μg/mL of the water solubility of the compound of formula 1. When diluted with physiological saline, the pH of the solution is close to neutral. Therefore, for intravenous administration, the risk of hemolysis and phlebitis caused by osmotic pressure and pH can be reduced.

Experimental Example 6: Evaluation of stability of beta cyclodextrin inclusion compound

The lyophilized power according to Example 9 was diluted in physiological saline, and then stored for 72 hours under different storage conditions. The presence or absence of precipitation was observed. The results are shown in Table 10.

[Table 10]

| Sample (72 hours after manufacture) | Peak area | Measurement concentration (mg/ml) |
|---|---|---|
| Cold storage | 1206769 | 2.92 |
| Storage in accelerated condition | 1249405 | 3.03 |
| Storage in long-term condition | 1252358 | 3.03 |

As shown in Table 10, it can be seen that the concentration of the compound of formula 1 is kept constant regardless of the storage conditions. It indicates that it is physically stable without precipitation even after dilution.

In addition, for intravenous administration (IV infusion), the composition of Example 9 was diluted 50-fold and 100-fold with physiological saline, and then the change in content was observed in order to evaluate physical stability, that is, whether precipitation occurs or not, depending on the storage time at room temperature. The results are shown in Tables 11 and 12.

[Table 11]

| 50-fold dilution | Immediately after manufacture | 4 hours after manufacture | | |
|---|---|---|---|---|
| | | Upper layer | Middle layer | Bottom layer |
| Peak area | 25314 | 25059 | 2581 5 | 25708 |
| Concentration (μg/ml) | 61.6 | 61 | 62.8 | 62.5 |

[Table 12]

| Sample (100-fold dilution) | Immediately after manufacture | 4 hours after manufacture | | |
|---|---|---|---|---|
| | | Upper layer | Middle layer | Lower layer |
| Peak area | 12968 | 12501 | 12851 | 12445 |
| Concentration (μg/ml) | 31.5 | 30.4 | 31.3 | 30.3 |

In addition, the composition of Example 9 was diluted 50-fold and then allowed to stand for a week. The content of the compound of formula 1 was measured, and the results are shown in Table 13.

[Table 13]

| Sample (1 week after manufacture) | Peak area | % content |
|---|---|---|
| Standard | 1090155 | - |
| Sample 1 | 1078759 | 99 |
| Sample 2 | 1076296 | 99.8 |
| Sample 3 | 1062600 | 98.7 |

As shown in Tables 11 to 13, the composition of Example 9 was diluted 50-fold and 100-fold and then allowed to stand at room temperature for 4 hours and for 1 week, respectively, and the content measured was uniformly maintained in the upper layer, the middle layer, and the lower layer. It indicates that it is physically stable without re-precipitation even after dilution.

In addition, the composition according to Example 9 in a glass vial and sealed. A stability test was conducted up to 24 weeks under accelerated condition (40 degrees/75% humidity), and the results are shown in Table 14.

[Table 14]

| Period (week) | Production rate (%) | |
|---|---|---|
| | Compound of formula 1 | Related substance 1 |
| Initial | 99.994 | 0.006 |

(continued)

| Period (week) | Production rate (%) | |
|---|---|---|
| | Compound of formula 1 | Related substance 1 |
| 1 | 99.99 | 0.01 |
| 2 | 99.99 | 0.011 |
| 4 | 99.99 | 0.011 |
| 8 | 99.992 | 0.004 |
| 12 | 99.992 | 0.004 |
| 16 | 99.992 | 0.004 |
| 24 | 99.985 | 0.012 |

As shown in Table 14, no significant changes were observed in the content and related substances until 6 months of acceleration. From this, it was found that the lyophilized powder according to Example 9 was physicochemically very stable.

Experimental Example 7: Evaluation of inhibition against MRSA strains

In order to verify the inhibitory effect of the compound of formula 1 on antibiotic-resistant strains, drug susceptibility was evaluated by treating the compound of formula 1 with *Staphylococcus aureus* phenotype isolated from each patient. In an in vitro test for antibiotic development, the most important result can be $MIC_{90}$ (minimum inhibitory concentration required to inhibit the growth of 90% of the total bacterial population). Table 15 shows the results of $MIC_{90}$ test with representative drugs which are currently on the market as a control drug, for about 100 methicillin-susceptible strains and about 100 MRSA strains which is currently socially problematic, which is carried out in the laboratory of Dr. Peter C. Appelbaum at Hershey Hospital who is recognized for its authority in the field of anti-infection worldwide.

[Table 15]

| **Drug** | **Methicillin-susceptible (μ g/mL, n=103)** | | | **Methicillin-resistant (μ g/mL, n=100)** | | |
|---|---|---|---|---|---|---|
| | Range | **$MIC_{50}$** | **$MIC_{90}$** | **Range** | **$MIC_{50}$ $MIC_{90}$** | |
| Compound of formula 1 | 0.06 - 1.0 | 0.25 | 0.25 | 0.06 - 1.0 | 0.25 | 0.25 |
| Vancomycin | 1.0 - 2.0 | 1 | 2 | 1.0 - >64.0 | 1 | 2 |
| Teicoplanin | 0.125 - 8.0 | 1 | 2 | 0.25 - >64.0 | 1 | 2 |
| Linezolid | 0.25 - 2.0 | 1 | 2 | 0.25 - 2.0 | 1 | 2 |
| Quinupristin dalfopristin | 0.25 - 2.0 | 1 | 2 | 0.25 - 2.0 | 1 | 2 |
| Daptomycin | 0.25 - 2.0 | 1 | 1 | 0.25 - 4.0 | 0.5 | 0.5 |
| Amoxicillin-clavulanate | 0.125 - 4.0 | 1 | 2 | 0.5 - >64.0 | >64,0 | >64.0 |
| Azithromycin | 0.25 - >64.0 | 1 | >64.0 | 0.5 - >64.0 | >64.0 | >64.0 |
| Levofloxacin | ≦0.06 - 32.0 | 0.25 | 4 | 0,125 - >32.0 | 1 | >32.0 |

As shown in Table 15, it is found that the $MIC_{90}$ value is 0.25 μg/mL irrespective of susceptible strains and non-susceptible strains, i.e., MRSA strains, which indicates 2 times to several ten times superior results compared to the control drugs. In particular, these strains include vancomycin-intermediate Staphylococcus aureus (VISA) strain which is resistant to vancomycin and vancomycin-resistant Staphylococcus aureus (VRSA) strain which is a super bacterium (vancomycin MIC > 64 μg/mL). From these results, it can be seen that the compound of formula 1 can be used as an effective therapeutic agent for diseases or disorders caused by bacterial infection, compared to conventional drugs such as vancomycin, teicoplanin, linezolid, amoxicillin-clavulanate, daptomycin, etc. Specifically, although not limited thereto, it may be usefully used as a therapeutic agent for bacterial infections related to diseases including urinary tract, respiratory tract, skin tissue infection, sepsis, and the like.

Experimental Example 8: Pharmacokinetic and pharmacodynamic analysis in mouse model

For the compound of formula 1, pharmacokinetic/pharmacodynamic experiments were conducted using a mouse infection model. To this end, experiments were conducted with Staphylococcus aureus ATCC 29213 (MSSA, standard strain) and 13B-382 (MRSA, clinical strain). As a medium, Mueller-Hinton broth or Cation-adjusted Mueller-Hinton broth was used. For the susceptibility test (minimum inhibitory concentration (MIC)), the compound of formula 1 was used.

An aseptic (Specific pathogen free, SPF) female, 6 weeks old (23 ~ 27g) ICR mouse (Orient Bio Inc, Gapyeong, Korea) was used, and the experiment was carried out in compliance with the regulations and procedures with the permission of the Ethics committees for animal experiments in accordance with the Animal Protection Act and the Laboratory Animal Act. Cyclophosphamide (Bexter, Frankfurt, Germany) was injected subcutaneously to induce reduction in neutrophils (<100/$mm^3$). Before the experiment, the test strain was incubated in Muller Hinton II broth for 24 hours at 37°C to obtain a concentration of $10^8$ CFU/mL. Then, it was diluted with physiological saline. 0.1 ml of the solution was inoculated into the thigh of the mouse (inoculation amount 1.0 x $10^5$ CFU/mL). After 2 hours, oral administration of the compound of formula 1 was started. A drug was administered every 3, 6, 12 and 24 hours at a dose of 7.5 mg to 240 mg/kg/day.

After 24 hours of drug administration, the mouse was euthanized with carbon dioxide gas and its thigh was separated, put in physiological saline, and cut finely with a homogenizer (Kinematica AG/ Polytron®). It was diluted 10 times, spread on Muller Hinton II broth, incubated at 37 °C for 24 hours. The number of viable cells was counted and recorded. The results were expressed as $\log_{10}$ CFU/thigh, and the measurement limit of the number of viable cells in the laboratory was $1 \times 10^2$ CFU/thigh.

T/MIC was evaluated as an index to determine the effect of antibiotics in combination with the antimicrobial action and pharmacokinetic results according to the antimicrobial dosage and administration. The T/MIC value represents the percentage of a dosage interval in which the serum level exceeds the MIC. The results are shown in Fig. 5. As shown in Fig. 5, it is found that as the T/MIC value increases, the effect of eradicating bacteria increases rapidly. When the T/MIC value was approximately 20% or higher, more than 99.9% of bacteria were eradicated. In addition, it is found that when the values of $AUC_{0-24h}$/MIC and $C_{max}$/MIC are increased, the effect of eradicating bacteria such as MRSA strains also increases rapidly.

In addition, it is found that the compound of formula 1 has the MIC value for Staphylococcus aureus ATCC 29213 and 13B-382 of 0.25 μg/mL, regardless of the strain. This value is lower than those of Oxacillin (0.25 and 16 μg/mL) and Vancomycin (0.5 and 1 μg/mL).

As described above, it is confirmed that the present invention can be effectively applied to the treatment of multidrug resistant bacterial infections.

The above descriptions are merely illustrative of the technical idea of the present invention, and those of ordinary skill in the technical field to which the present invention pertains can make various modifications and variations without departing from the essential characteristics of the present invention. In addition, the embodiments disclosed in the present invention are not intended to limit the technical idea of the present invention, but to explain the technical idea, and the scope of the technical idea of the present invention is not limited by these embodiments. The scope of protection of the present invention should be interpreted by the appended claims, and all technical ideas within the scope equivalent thereto should be interpreted as being included in the scope of the present invention.

## Claims

**1.** A composition for injection comprising:

1-(3-amino-2-methylbenzyl)-4-(2-thiophen-2-yl-ethoxy)-1H-pyridin-2-one or a salt thereof; and
a polymer compound, a solubilizing agent, or a mixture thereof.

**2.** The composition for injection according to claim 1, wherein based on the total weight of the composition, the content of 1-(3-amino-2-methylbenzyl)-4-(2-thiophen-2-yl-ethoxy)-1H-pyridin-2-one or a salt thereof is 0.1 to 10% by weight, the content of the polymer compound is 5 to 40% by weight, and the content of the solubilizing agent is 10 to 30% by weight.

**3.** The composition for injection according to claim 1, wherein the polymer compound comprises one or more selected from the group consisting of dextrin, cyclodextrin, poloxamer, dextran, pectin, pectin derivatives, alginate, starch, hydroxypropyl methylcellulose, hydroxypropylcellulose, hydroxymethylcellulose, hydroxylethylcellulose, methylcellulose, sodium carboxymethyl cellulose, hydroxypropyl methylcellulose acetate succinate, hydroxylethylmethyl cellulose, guar gum, locust bean gum, tragacantha, carrageenan, acacia gum, arabic gum, gellan gum, xanthan gum, gelatin, casein, polyvinyl alcohol, polyvinyl pyrrolidone, polyvinyl acetaldiethyl aminoacetate, poly(butylmethacrylate,(2-dimethylaminoethyl)methacrylate,methylmethacrylate) copolymer, polyethylene glycol, polyethylene oxide and carbomer.

4. The composition for injection according to claim 1, wherein the solubilizing agent comprises one or more selected from the group consisting of propylene glycol, polyethylene glycol, dipropylene glycol, diethylene glycol, diethylene glycol monoethyl ether, glycerol, Tween 80, cremophor and transcutol.

5. The composition for injection according to claim 1, wherein the composition is in the form of a liquid, emulsion or lyophilized powder.

6. A method of preparing a composition for injection comprising 1-(3-amino-2-methylbenzyl)-4-(2-thiophen-2-yl-ethoxy)-1H-pyridin-2-one or a salt thereof, the method comprising:

adding 4-benzyloxy-1H-pyridone, 2-methyl-3-nitro-benzylchloride and potassium tert-butoxide to dimethylformamide and mixing and reacting them under heating;
reacting the mixture, adding purified water and drying under heating;
dissolving the dried product in an organic solvent and adding purified water for layer separation;
recovering the organic layer, filtering and concentrating to prepare a concentrate;
reconcentrating the concentrate and adding hexane to prepare an intermediate precipitate;
dissolving the obtained precipitate, cooling, filtering and drying to obtain a dried product; and
dissolving the obtained dried product in an organic solvent and then adding and reacting iron chloride hexahydrate, activated carbon and hydrazine monohydrate, cooling and filtering to obtain a final precipitate, and drying and pulverizing the obtained final precipitate to prepare the compound.

7. The method of preparing a composition for injection according to claim 6, wherein the method further comprises adding an acidic substance.

8. The method of preparing a composition for injection according to claim 7, wherein the acidic substance comprise one or more selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, oxalic acid, fumaric acid, malonic acid, maleic acid, methanesulfonic acid, benzenesulfonic acid, p-toluenesulfonic acid, naphthalenesulfonic acid and EDTA.

9. The method of preparing a composition for injection according to claim 6, wherein the method further comprises:

1) dissolving a polymer compound and 1-(3-amino-2-methylbenzyl)-4-(2-thiophen-2-yl-ethoxy)-1H-pyridin-2-one or a salt thereof in a solvent; and
2) vacuum drying the solution of 1) and then micronizing the resulting solid.

10. The method of preparing a composition for injection according to claim 6, wherein the method further comprises:

1) dissolving a polymer compound and a lipid-based surfactant and 1-(3-amino-2-methylbenzyl)-4-(2-thiophen-2-yl-ethoxy)-1H-pyridin-2-one or a salt thereof in a solvent;
2) gradually adding a solubilizing agent to the solution of 1); and
3) centrifugating and vacuum drying the solution of 3) and then homogenizing it.

11. The method of preparing a composition for injection according to claim 10, wherein the lipid-based surfactant includes soybean oil.

12. The method of preparing a composition for injection according to claim 6, wherein the composition is prepared in the form of a solid dispersion, a liposome formulation, or a combination thereof.

13. The method of preparing a composition for injection according to claim 1, wherein the composition is for the treatment of bacterial infections.

[Fig. 1]

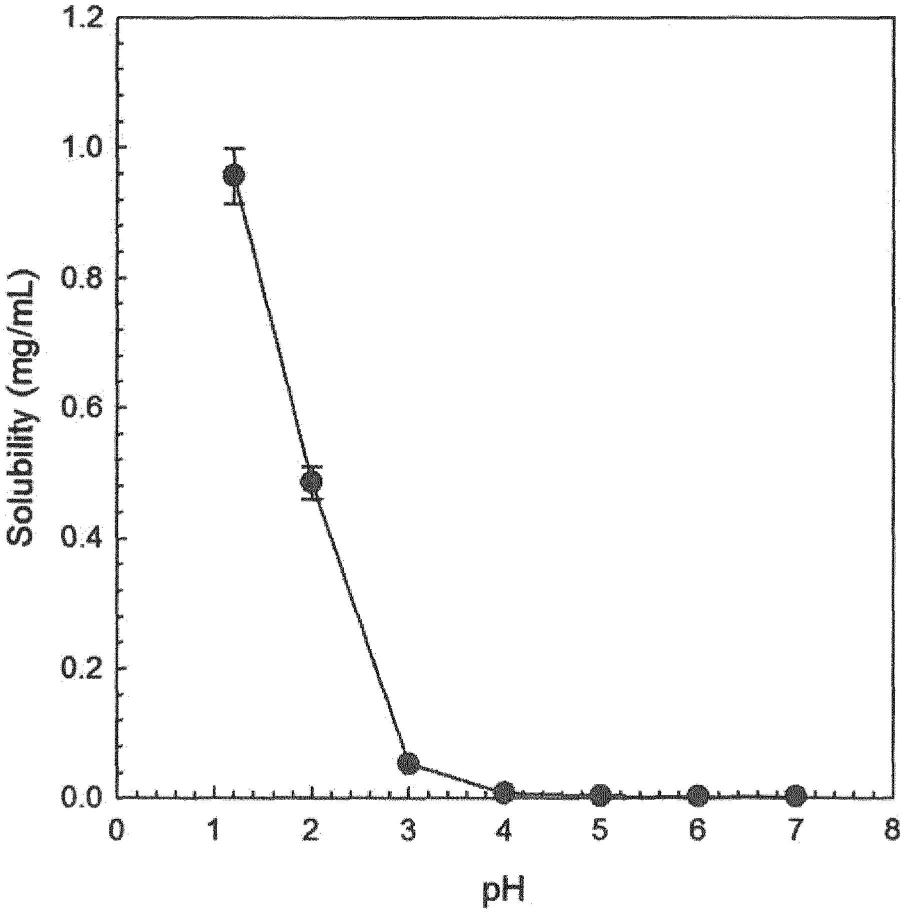

[Fig. 2]

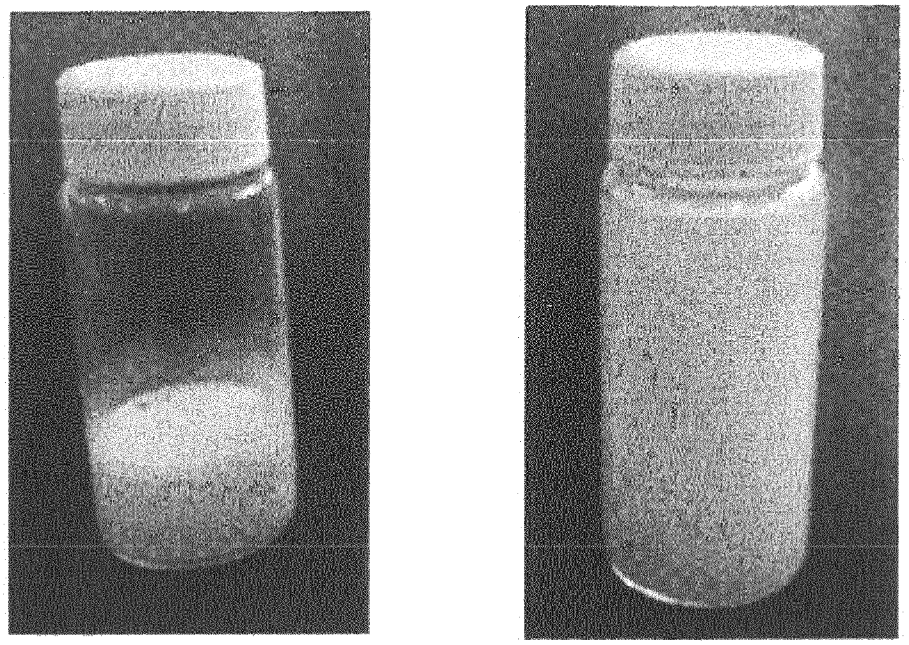

Undiluted: Precipitates X 20-fold diluted: Precipitates X

[Fig. 3]

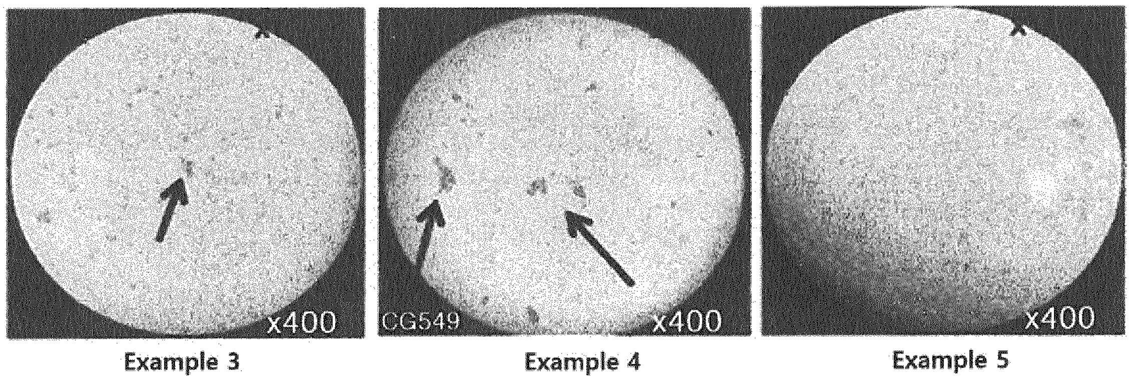


Example 3 Example 4 Example 5

[Fig. 4]

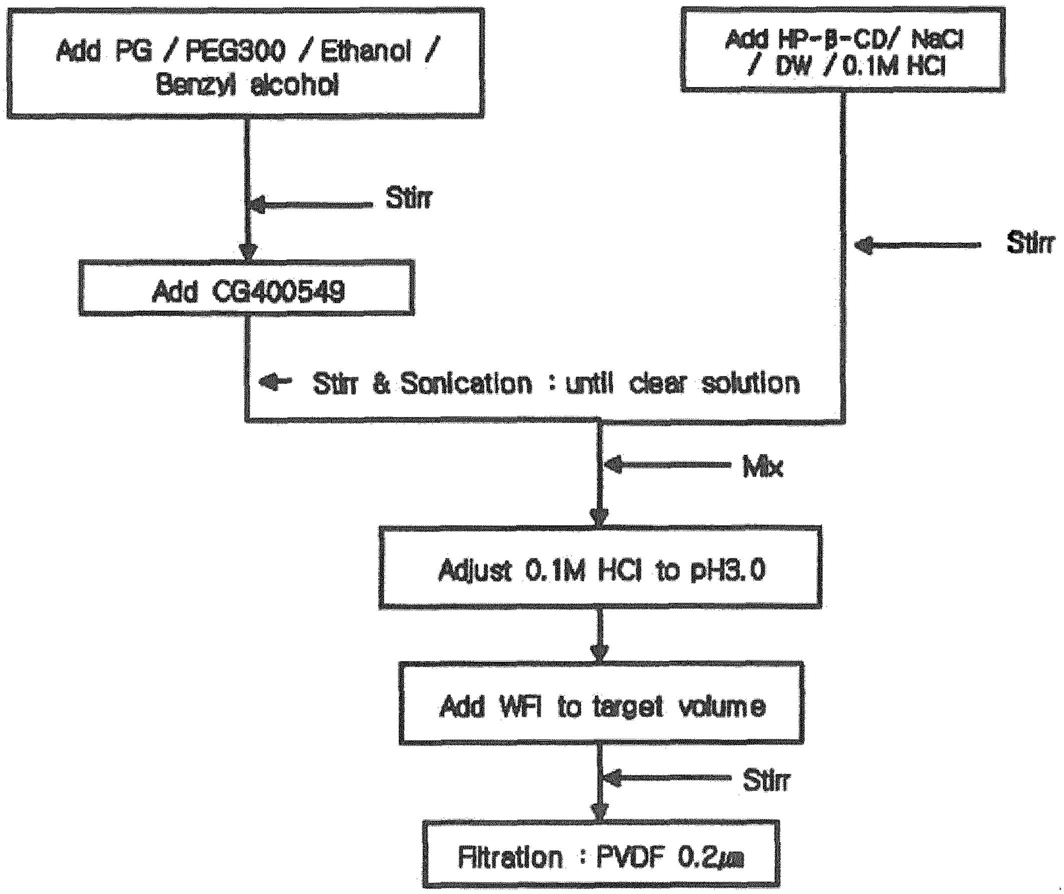
Add PG / PEG300 / Ethanol / Benzyl alcohol
Add HP-β-CD/ NaCl / DW / 0.1M HCl
Stirr
Stirr
Add CG400549
Stirr & Sonication : until clear solution
Mix
Adjust 0.1M HCl to pH3.0
Add WFI to target volume
Stirr
Filtration : PVDF 0.2㎛

[Fig. 5]

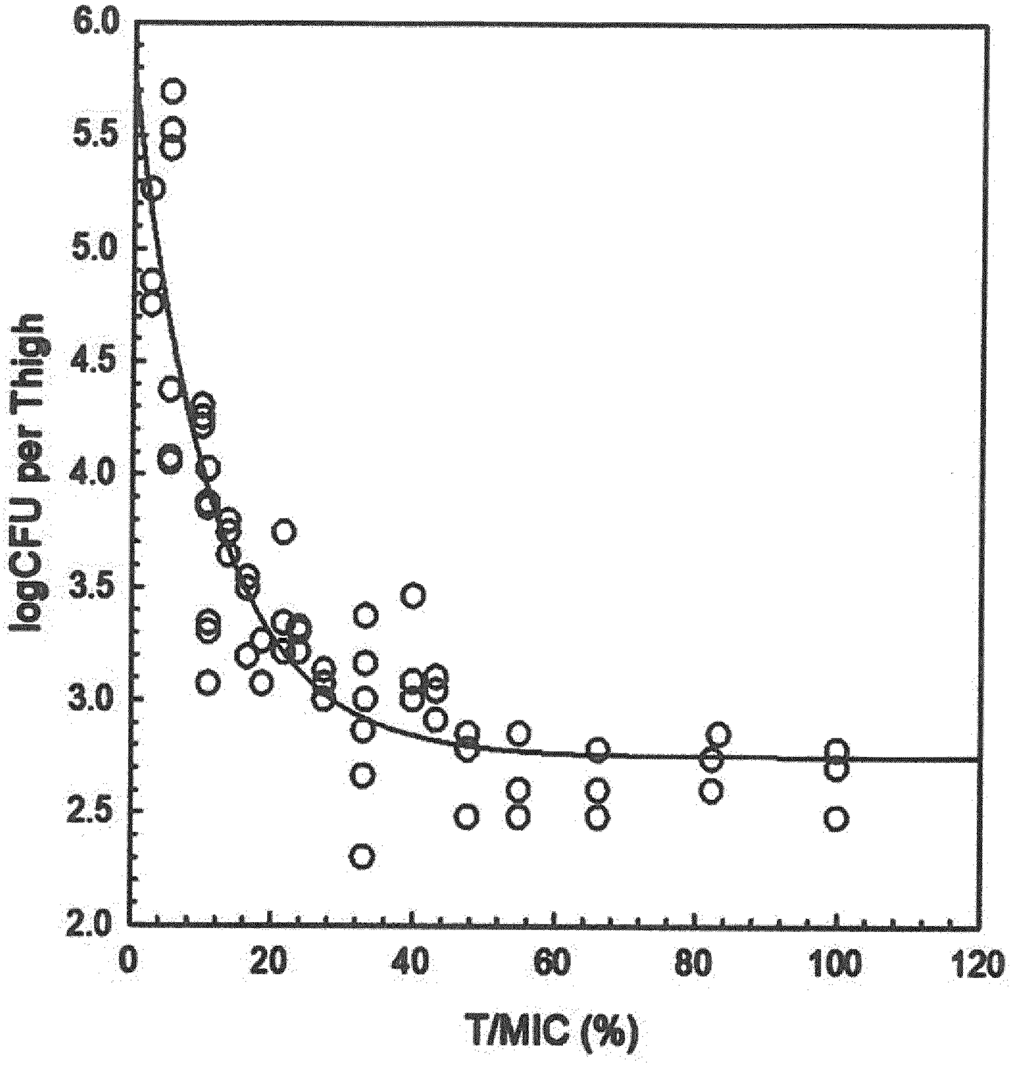
logCFU per Thigh
6.0
5.5
5.0
4.5
4.0
3.5
3.0
2.5
2.0
0
20
40
60
80
100
120
T/MIC (%)

**INTERNATIONAL SEARCH REPORT**

International application No.
**PCT/KR2020/001033**

A. CLASSIFICATION OF SUBJECT MATTER
***A61K 9/00(2006.01)i, A61K 9/19(2006.01)i, A61K 47/36(2006.01)i, A61K 47/40(2006.01)i, A61K 47/38(2006.01)i, A61K 47/32(2006.01)i, A61K 47/10(2006.01)i, A61K 31/4412(2006.01)i, A61P 31/04(2006.01)i***
According to International Patent Classification (IPC) or to both national classification and IPC

B. FIELDS SEARCHED

Minimum documentation searched (classification system followed by classification symbols)
A61K 9/00; A61K 314045; A61K 9/10; A61K 9/14; C07D 209/18; C07D 20914; C07D 471/04; A61K 9/19; A61K 47/36; A61K 47/40; A61K 47/38; A61K 47/32; A61K 47/10; A61K 31/4412; A61P 31/04

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: FAB I inhibitor, CG400549, polymer, solubilizing agent, injection, bacterial infection

C. DOCUMENTS CONSIDERED TO BE RELEVANT

| Category* | Citation of document, with indication, where appropriate, of the relevant passages | Relevant to claim No. |
|---|---|---|
| X | KR 10-2008-0068060 A (CRYSTALGENOMICS, INC.) 22 July 2008<br>See abstract; paragraphs [49], [352]-[353], [367]-[369], [802]; claims 1-27. | 1-13 |
| A | PARK, H. S. et al. Antistaphylococcal activities of CG400549, a new bacterial enoyl-acyl carrier protein reductase (FabI) inhibitor. Journal of antimicrobial chemotherapy. 2007, vol. 60, pages 568-574<br>See abstract; table 1; page 570. | 1-13 |
| A | BOGDANOVICH, T. et al. Antistaphylococcal activity of CG400549, a new experimental FabI inhibitor, compared with that of other agents. Antimicrobial agents and chemotherapy. 2007, vol. 51, no. 11, pages 4191-4195<br>See the entire document. | 1-13 |
| A | KR 10-2007-0112164 A (ELAN PHARMA INTERNATIONAL LTD.) 22 November 2007<br>See the entire document. | 1-13 |
| A | US 6762201 B1 (MILLER, W. H. et al.) 13 July 2004<br>See the entire document. | 1-13 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 JUNE 2020 (19.06.2020) | **19 JUNE 2020 (19.06.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2020/001033**

| Patent document cited in search report | Publication date | Patent family member | Publication date |
|---|---|---|---|
| KR 10-2008-0068060 A | 22/07/2008 | BR PI0617268 A2 | 19/07/2011 |
| | | CA 2625962 A1 | 19/04/2007 |
| | | CA 2625962 C | 04/11/2014 |
| | | CN 101282930 A | 08/10/2008 |
| | | CN 101282930 B | 24/10/2012 |
| | | EP 1948601 A1 | 30/07/2008 |
| | | EP 1948601 B1 | 13/04/2016 |
| | | ES 2576579 T3 | 08/07/2016 |
| | | JP 2009-511575 A | 19/03/2009 |
| | | JP 5049977 B2 | 17/10/2012 |
| | | KR 10-1502335 B1 | 16/03/2015 |
| | | KR 10-1522713 B1 | 26/05/2015 |
| | | KR 10-2014-0029550 A | 10/03/2014 |
| | | US 2007-0135465 A1 | 14/06/2007 |
| | | US 7973060 B2 | 05/07/2011 |
| | | WO 2007-043835 A1 | 19/04/2007 |
| KR 10-2007-0112164 A | 22/11/2007 | AU 2006-214443 A1 | 24/08/2006 |
| | | AU 2006-214443 B2 | 16/06/2011 |
| | | AU 2006-214443 C1 | 24/11/2011 |
| | | BR PI0608087 A2 | 10/11/2009 |
| | | CA 2597716 A1 | 24/08/2006 |
| | | CN 101189001 A | 28/05/2008 |
| | | EA 013433 B1 | 30/04/2010 |
| | | EA 200701731 A1 | 28/02/2008 |
| | | EP 1853234 A2 | 14/11/2007 |
| | | EP 2332524 A1 | 15/06/2011 |
| | | EP 2353590 A1 | 10/08/2011 |
| | | JP 2008-530134 A | 07/08/2008 |
| | | MX 2007009915 A | 06/11/2007 |
| | | NO 20074602 L | 31/10/2007 |
| | | US 2006-0198896 A1 | 07/09/2006 |
| | | US 2009-0304801 A1 | 10/12/2009 |
| | | WO 2006-088894 A2 | 24/08/2006 |
| | | WO 2006-088894 A3 | 30/11/2006 |
| | | ZA 200707610 B | 26/11/2008 |
| US 6762201 B1 | 13/07/2004 | AT 294578 T | 15/05/2005 |
| | | AU 1192501 A | 23/04/2001 |
| | | CO 5251401 A1 | 28/02/2003 |
| | | DE 60019954 T2 | 23/02/2006 |
| | | EP 1225895 A1 | 31/07/2002 |
| | | EP 1225895 B1 | 04/05/2005 |
| | | JP 2003-511415 A | 25/03/2003 |
| | | JP 4961084 B2 | 27/06/2012 |
| | | WO 01-26654 A1 | 19/04/2001 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20190007288


### Non-patent literature cited in the description


- **PAYNE et al.** *Drug Discovery Today,* 2001, vol. 6, 537-544